# FASCICULE DE BREVET EUROPEEN

(11) **EP 3 338 884 B1**
(45) Date de publication et mention de la délivrance du brevet: **15.07.2020**
(21) Numéro de dépôt: 17306800.8
(22) Date de dépôt: 18.12.2017
(51) Int. Cl.: B01J 19/24, B01J 19/00, F28G 9/00, F28D 15/00

(54) **PROCEDE D'OLIGOMERISATION D'OLEFINES METTANT EN OEUVRE UN DISPOSITIF DE NETTOYAGE**
OLIGOMERISIERUNGSVERFAHREN VON OLEFINEN UNTER VERWENDUNG EINER REINIGUNGSVORRICHTUNG
OLIGOMERIZATION METHOD OF OLEFINS USING A CLEANING DEVICE

(30) Priorité: 22.12.2016 FR 1663200
(43) Date de publication de la demande: 27.06.2018
(73) Titulaire: IFP Energies nouvelles, 92852 Rueil-Malmaison Cedex (FR); AXENS, 92508 Rueil-Malmaison Cedex (FR)
(72) Inventeur: BOUTROT, Catherine, 78400 CHATOU (FR); JANOT, Nicolas, 92150 SURESNES (FR); LIEGE, Xavier, 75004 PARIS (FR); NIDERKORN, Etienne, 92500 RUEIL-MALMAISON (FR); PIGOURIER, Jérôme, 92190 MEUDON (FR); VINEL, Daniel-Jean, 78130 LES MUREAUX (FR); FAVRE, Frédéric, 69005 LYON (FR); MAGNA, Lionel, 69007 LYON (FR)
(74) Mandataire: IFP Energies nouvelles

(56) Documents cités:
- WO-A1-2012/072178
- KR-A- 20020 050 861
- Peter R Pujadó ET AL: "Catalytic olefin condensation", Handbook of Petroleum Processing, 1 janvier 2006 (2006-01-01), pages 372-399, XP055375700, Springer Extrait de l'Internet: URL:http://ajaysingh.in/files/4313/8293/74 59/Chemical_Engineering_-_Oil_-_Handbook_O f_Petroleum_Processing.pdf [extrait le 2017-05-24]

## Description

L'invention concerne le domaine de l'oligomérisation de l'éthylène. Cette oligomérisation vise à produire des alphaoléfines utilisées comme co-monomère des procédés de production de polyéthylène. La réaction d'oligomérisation est le plus souvent réalisée dans un procédé de catalyse en phase liquide. Elle est par ailleurs fortement exothermique nécessitant un refroidissement externe via des échangeurs de chaleur. Les réactions secondaires de l'oligomérisation sont la production d'oligomères très longs et/ou de polymères. Ces polymères s'avèrent la plupart du temps encrassant, en particulier ils se déposent sur les tubes d'échangeurs de chaleur. Ceci nécessite des arrêts fréquents du procédé, pour isoler et nettoyer l'échangeur de chaleur encrassé, qui nuisent à l'efficacité et l'opérabilité du procédé et affectent son économie de production.

Les tentatives visant à atténuer les problématiques liées aux dépôts de polymères dans les équipements du procédé sont connues de l'homme du métier. Ces tentatives se sont traduites la plupart du temps par l'utilisation de divers moyens mécaniques comme par exemple le nettoyage par projection d'eau à haute pression. Cette méthode présente l'inconvénient de devoir ouvrir l'échangeur et de plus nécessite un séchage poussé de ce dernier avant sa remise en service, l'humidité étant un poison connu de ce type de catalyse.

WO 2012/072178 A1 divulgue une méthode de nettoyage par circulation de solvant des équipements d'une installation d'oligomérisation de l'éthylène en alpha-oléfines
KR 2002 0050861 A décrit une méthode de nettoyage d'échangeurs de chaleur comprenant deux échangeurs de chaleur permutables.

Le nettoyage par un solvant est possible, le polyéthylène étant, aux conditions de température adéquates soluble dans la plupart des hydrocarbures légers. Le solvant ayant servi au nettoyage est alors chargé en polymères et oligomères lourds ainsi qu'en catalyseur (ou composés issus du catalyseur, tel que les métaux). Par conséquent cela présente l'inconvénient de nécessiter un apport externe en solvant outre l'investissement en moyens permettant la séparation des polymères, des oligomères lourds et catalyseurs (ou composés issus du catalyseur) d'une part et du solvant d'autre part.

La demanderesse dans ses recherches a mis au point un nouveau procédé d'oligomérisation de l'éthylène plus efficace et qui nécessite moins d'arrêts pour le nettoyage des équipements grâce à la mise en œuvre intégrée d'un procédé de nettoyage desdits équipements du procédé, voire des échangeurs de chaleur mis en œuvre dans lesdits procédés de type oligomérisation des oléfines.

### Résumé de l'invention

L'invention concerne un procédé d'oligomérisation de l'éthylène en alphaoléfines, selon la revendication 1, comprenant
- une étape d'oligomérisation de l'éthylène dans une section réactionnelle (1) comprenant un réacteur (10) en présence d'un catalyseur, éventuellement un diluant,
- une étape de désactivation du catalyseur contenu dans l'effluent réactionnel,
- une étape d'évaporation des produits contenus dans l'effluent réactionnel, pour les séparer du catalyseur désactivé, réalisée dans une section d'évaporation (2),
- une étape de séparation des produits contenus dans l'effluent réactionnel 103 dans une section de séparation (3),
ledit réacteur étant muni d'au moins une boucle de refroidissement (100 a/b, 101 a/b, 102 a/b) au moyen de laquelle on fait circuler le milieu réactionnel à travers au moins deux échangeurs de chaleur permutables aptes à être connectés à au moins une boucle de refroidissement, de sorte que lorsque au moins un échangeur de chaleur est en fonctionnement connecté à au moins une boucle de refroidissement, l'autre échangeur de chaleur déconnecté est soumis à une étape de nettoyage au moyen d'un dispositif de nettoyage dans lequel on fait circuler en boucle un solvant apte à nettoyer ledit échangeur de chaleur, le dispositif de nettoyage comprenant :
- un ballon d'inventaire de solvant de nettoyage (40),
- un échangeur de chaleur (43) permettant de chauffer le solvant de nettoyage à une température supérieure à 130°C de façon à permettre la dissolution du polymère déposé dans l'échangeur de chaleur déconnecté,
- une pompe de recirculation (42) permettant la circulation en boucle du solvant de nettoyage dans le dispositif de nettoyage entre le ballon d'inventaire de solvant (40), l'échangeur de chaleur (43) du solvant et l'échangeur de chaleur déconnecté que l'on souhaite nettoyer.
dans lequel au moins une partie du solvant de nettoyage chargé (406, 407) est envoyée en mélange avec une partie de l'effluent réactionnel 103 dans la section d'évaporation (2), soit en amont (213), soit en aval (211) d'un échangeur de chaleur (20) de ladite section d'évaporation (2) lorsque cet échangeur de chaleur est présent.

Un des avantages du procédé selon l'invention est de fournir un moyen permettant un nettoyage rapide et efficace des équipements du procédé, en particulier des échangeurs mis en œuvre, sans nuire à la rentabilité et l'opérabilité du procédé d'oligomérisation.

Un autre avantage du procédé est d'être d'une mise en œuvre facile et intégrée avec les différentes étapes du procédé, notamment par l'utilisation de solvant de nettoyage à partir du procédé lui-même.

Le procédé selon l'invention permet de corriger les inconvénients de l'art antérieur en minimisant l'investissement matériel et/ou la perte en productivité et/ou en minimisant la consommation de solvant de nettoyage. Il permet le lavage de l'échangeur de chaleur par dissolution dans un solvant hydrocarbure des polymères formés et déposés sur les parois de l'échangeur de chaleur. Il permet aussi d'intégrer l'élimination des oligomères lourds et/ou polymères et/ou catalyseurs usés contenus dans le solvant de nettoyage dans la section de désactivation du catalyseur et d'évaporation par mélange d'au moins une partie du solvant de nettoyage chargé en oligomères lourds et/ou polymères et/ou catalyseurs usés avec l'effluent de la section d'oligomérisation.

### Description sommaire des figures

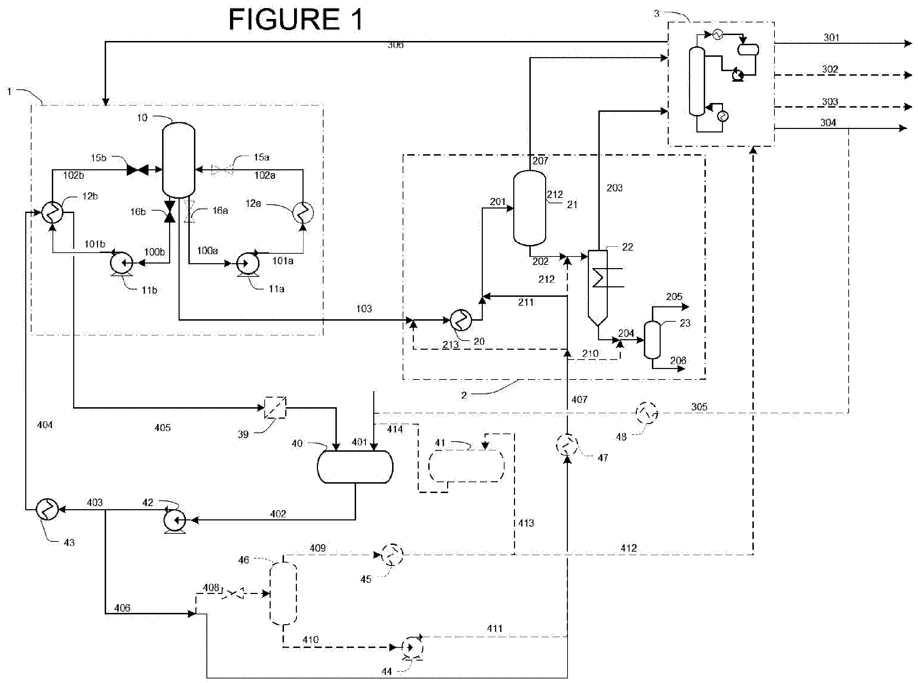

La figure 1 décrit un schéma de réalisation du procédé mis en œuvre avec le dispositif de nettoyage selon l'invention lorsqu'un échangeur de chaleur est en fonctionnement (12a) et que l'autre est en nettoyage (12b) avec le dispositif de nettoyage.

### Description détaillée de l'invention

L'invention concerne un procédé d'oligomérisation de l'éthylène en alphaoléfines comprenant
- une étape d'oligomérisation de l'éthylène dans une section réactionnelle (1) comprenant un réacteur (10) en présence d'un catalyseur, éventuellement un diluant,
- une étape de désactivation du catalyseur contenu dans l'effluent réactionnel (103),
- une étape d'évaporation des produits contenus dans l'effluent réactionnel, pour les séparer du catalyseur désactivé, réalisée dans une section d'évaporation (2),
- une étape de séparation des produits contenus dans l'effluent réactionnel dans une section de séparation (3),
ledit réacteur étant muni d'au moins une boucle de refroidissement (100 a/b, 101 a/b, 102 a/b) au moyen de laquelle on fait circuler le milieu réactionnel à travers au moins deux échangeurs de chaleur permutables aptes à être connectés à au moins une boucle de refroidissement, de sorte que lorsque au moins un échangeur de chaleur est en fonctionnement connecté à au moins une boucle de refroidissement, l'autre échangeur de chaleur déconnecté est soumis à une étape de nettoyage au moyen d'un dispositif de nettoyage dans lequel on fait circuler en boucle un solvant apte à nettoyer ledit échangeur de chaleur, le dispositif de nettoyage comprenant :
- un ballon d'inventaire du solvant de nettoyage (40),
- un échangeur de chaleur (43) permettant de chauffer le solvant de nettoyage à une température supérieure à 130°C de façon à permettre la dissolution du polymère déposé dans l'échangeur de chaleur déconnecté,
- une pompe de recirculation (42) permettant la circulation en boucle du solvant de nettoyage dans le dispositif de nettoyage entre le ballon d'inventaire du solvant de nettoyage (40), l'échangeur de chaleur (43) du solvant de nettoyage et l'échangeur de chaleur déconnecté que l'on souhaite nettoyer,
dans lequel au moins une partie du solvant de nettoyage chargé (406, 407) est envoyée en mélange avec une partie de l'effluent réactionnel 103 dans la section d'évaporation (2), soit en amont (213), soit en aval (211) d'un échangeur de chaleur (20) de ladite section d'évaporation (2) lorsque cet échangeur de chaleur est présent.

Selon l'invention, au moins une partie du solvant de nettoyage issu de la boucle de solvant du dispositif de nettoyage (406, 407) est envoyée dans l'étape d'évaporation des produits dans la section d'évaporation (2).

Pour des raisons de clarté, l'invention sera, dans la suite du texte, exposée en référence à la figure 1 qui décrit un schéma illustratif du procédé. En aucun cas, la référence à la figure 1 ne saurait être limitative de la portée du procédé.

Le solvant de nettoyage ayant servi au nettoyage, contenant des oligomères lourds et/ou du polymère et/ou du catalyseur usé est appelé solvant de nettoyage chargé dans la suite du texte.

### Section réactionnelle (1)

Conformément à l'invention, le procédé d'oligomérisation de l'éthylène en alphaoléfines comprend une étape d'oligomérisation de l'éthylène réalisée dans une section réactionnelle (1) comprenant un réacteur (10) en présence d'un catalyseur. Avantageusement, la réaction d'oligomérisation de l'éthylène est un procédé de catalyse en phase liquide, généralement homogène, en présence ou non d'un diluant, par un catalyseur de type Ziegler qui comprend généralement un composé d'un métal tel que le titane, le chrome, le zirconium et au moins un composé organoaluminique. La réaction est de préférence réalisée à une température comprise entre la température ambiante et 200°C, de préférence entre 30 et 170°C et à une pression comprise entre 0,5 et 20 MPa, de préférence entre 1,0 et 10 MPa. Cette réaction d'oligomérisation est parfois réalisée dans un diluant inerte tel que l'ortho-xylène ou le cyclohexane.

Dans le cadre général de l'invention, le procédé d'oligomérisation de l'éthylène peut être un procédé de dimérisation de l'éthylène en butène-1, un procédé de trimérisation de l'éthylène en hexène-1 ou un procédé de tétramérisation de l'éthylène en octène-1.

Les réactions d'oligomérisation de l'éthylène sont exothermiques. La chaleur générée par la réaction doit être extraite afin d'éviter une élévation incontrôlée de la température du milieu réactionnel. Selon le niveau de température atteint, les conséquences sont une perte de la sélectivité par dégradation thermique du catalyseur et des produits de la réaction. La chaleur de réaction générée dans le réacteur est extraite en utilisant au moins une boucle de refroidissement (conduites 100a/b, 101a/b, 102a/b) comprenant au moins un échangeur de chaleur (12a/b).

L'effluent réactionnel 103 issu de la section réactionnelle (1) d'oligomérisation de l'éthylène comprend de l'éthylène non converti, des alphaoléfines telles que le butène-1, l'hexène-1, l'octène-1, et d'autres produits de réaction de C4 à C30+ (c'est-à-dire comportant entre 4 et plus de 30 atomes de carbone par molécule), ainsi qu'un éventuel diluant de la réaction d'oligomérisation. Ledit effluent comprend aussi au moins en partie du catalyseur. L'effluent réactionnel (103) peut être circulé dans un échangeur de chaleur (20) dans lequel il est vaporisé dans la section d'évaporation (2).

Dans le cas particulier d'un procédé de dimérisation de l'éthylène, cette vaporisation dans l'échangeur de chaleur (20) est de préférence réalisée à une pression égale ou sensiblement identique à celle de l'étape de dimérisation de l'éthylène et à la température de 80°C, permettant dans la section d'évaporation (2), notamment dans un ou des ballons flash de la section d'évaporation (2), la séparation d'au moins une phase gazeuse et d'au moins une phase liquide.

### Section de désactivation et d'évaporation (2)

Conformément à l'invention, de manière générale, à la sortie de la section réactionnelle (1) d'oligomérisation, le catalyseur contenu dans l'effluent (103) est soumis à une étape de désactivation réalisée, de préférence en amont de la section d'évaporation (2). Cette désactivation est réalisée par tout composé connu de l'homme du métier pour son activité inhibitrice du catalyseur d'oligomérisation. Par exemple, un composé polaire oxygéné ou azoté destiné à désactiver sensiblement la totalité du catalyseur présent dans l'effluent 103. De tels inhibiteurs de catalyseurs azotés sont par exemple décrits dans le brevet européen EP-B-200654.

L'inhibiteur du catalyseur utilisé dans l'étape de désactivation est généralement sélectionné parmi les amines, de préférence les amines primaires ou secondaires de formule générale R₁R₂NH dans laquelle R₁ est l'hydrogène ou un radical hydrocarboné et R₂ est un radical hydrocarboné, ou les alcools linéaires ou ramifiés ayant de préférence de 2 à 20 atomes de carbone, de manière plus préférée de 5 à 15 atomes de carbone ou des acides carboxyliques tels que l'acide n-octanoïque ou l'eau ou l'ammoniaque.

Lorsque l'inhibiteur du catalyseur est sélectionné parmi les amines on utilisera de manière préférée un inhibiteur sélectionné parmi les composés suivants ou leurs mélanges : cyclohexylamine, éthyl-2-hexylamine, arylamine, stéarylamine, oleylamine, aniline, N-methyl aniline, dibutylamine, didecylamine, les mélanges d'amines obtenus à partir de corps gras naturels tels que le suif, l'huile de palme ou l'huile de coprah.

Lorsque l'inhibiteur du catalyseur est sélectionné parmi les alcools, on utilisera un alcool linéaire ou ramifié de 2 à 20 atomes de carbone, comme par exemple le 1-hexanol, 2-hexanol, 3-hexanol, 2-methyl-1-hexanol, 2-ethyl-1-hexanol, 1-heptanol, 2- heptanol, 2-methyl-3-heptanol, 1-octanol, 2-octanol, 3-octanol, 1-decanol, 2-decanol, 3-decanol, 2 ethyl-1-decanol ; un diol comme par exemple l'éthylèneglycol, les propanediols, les butanediols ou encore un polyol comme le glycérol, utilisés seuls ou en mélange. De manière préférée, on utilisera le 2-ethyl-1-hexanol.

Lorsque l'inhibiteur du catalyseur est sélectionné parmi les acides carboxyliques on utilisera un acide linéaire ou ramifié de 2 à 20 atomes de carbone, comme par exemple l'acide acétique, l'acide propionique, l'acide butyrique, l'acide pentanoique (valérique), l'acide hexanoïque, l'acide 2-ethylhexanoique, l'acide octanoïque (caprylique), l'acide dodécanoïque, l'acide stéarique, l'acide benzoïque, l'acide 2-hydroxybenzoïque, utilisés seuls ou en mélange. De manière préférée, on utilisera l'acide 2-ethylhexanoique et l'acide octanoïque (caprylique).

Après l'étape de désactivation du catalyseur, selon l'invention, l'effluent réactionnel issu du réacteur désactivé (103) (étape de désactivation non représentée dans la figure 1), est soumis à une étape d'évaporation pour séparer d'une part le catalyseur désactivé ainsi que les sous-produits lourds de la réaction et d'autre part l'éthylène non converti, les produits formés ainsi que le diluant éventuel. Cette étape d'évaporation est de préférence réalisée par vaporisation d'une partie de l'effluent réactionnel de façon à obtenir une ou plusieurs phases vapeurs contenant l'éthylène non converti, les produits formés et l'éventuel diluant. Cette ou ces phases vapeurs sont avantageusement envoyées (voir conduites 203, 207) vers la section (3) de séparation dans laquelle les différents produits d'intérêt formés sont séparés entre eux.

Conformément à l'invention, dans l'étape d'évaporation des produits contenus dans l'effluent réactionnel 103 réalisée dans la section d'évaporation (2) on sépare le catalyseur de l'ensemble des produits de la réaction.

Dans une variante du procédé de l'invention, la section d'évaporation (2) peut comprendre au moins un ballon flash (21, 23) (ballons/séparateurs avec équilibre liquide-vapeur) et au moins un vaporisateur ou évaporateur à film mince ou à film tombant (22) permettant de vaporiser le produit recherché.

Dans le cas particulier d'un procédé de dimérisation de l'éthylène, l'effluent réactionnel 103, de préférence issu de l'échangeur de chaleur (20), seule ou mélangée avec au moins une partie du solvant de nettoyage issu du flux 406-407 (flux 211, 201), peut être vaporisé dans un premier ballon flash 21 dans des conditions permettant de séparer une phase gazeuse (207) contenant essentiellement du butène-1 et d'autres produits hydrocarbonés en C6 et C8 et de l'éthylène et une seconde phase liquide (202) contenant le catalyseur désactivé. Il est possible dans un mode de réalisation qu'au moins une partie du solvant de nettoyage issu du flux 406-407 (flux 213) soit envoyée en mélange avec l'effluent (103) en amont de l'échangeur de chaleur (20). Avantageusement, au moins une partie de la phase liquide (202), seule ou mélangée avec au moins une partie du solvant de nettoyage issu du flux 406-407 (flux 212) peut ensuite être dirigée vers un vaporiseur ou évaporateur à film mince (22) dans lequel elle est évaporée dans des conditions permettant la séparation d'une phase gazeuse (203) contenant essentiellement du butène-1 et éventuellement des produits hydrocarbonés plus lourds, et d'une phase liquide enrichie en catalyseur désactivé concentré (204). Le taux d'évaporation dans l'évaporateur à film mince ou à film tombant est généralement de l'ordre de 99% poids par rapport à la phase liquide entrant dans l'évaporateur. Au moins une partie de la phase liquide enrichie en catalyseur désactivé concentré (204) seule ou mélangée avec au moins une partie du solvant de nettoyage issu du flux 406-407 (flux 210) est avantageusement détendue et flashée dans un ballon flash (23) générant une phase vapeur (205) sensiblement dépourvue de polymère et catalyseur désactivé et une phase liquide (206) concentrée en polymères et catalyseur désactivé (206). Avantageusement, les phases gazeuses (207, 203) issues de l'étape d'évaporation réalisée dans la section d'évaporation (2) et contenant essentiellement du butène-1 et de l'éthylène ainsi que des produits hydrocarbonés plus lourds sont condensées sous la forme d'un produit liquide qui est envoyé dans l'étape de séparation réalisée dans la section de séparation (3) dans laquelle les différents produits seront séparés entre eux.

Dans le cas particulier d'un procédé de trimérisation de l'éthylène, l'effluent réactionnel du réacteur est de préférence envoyé dans la section de désactivation et d'évaporation (2) comprenant avantageusement des ballons flash. Le catalyseur dans l'effluent réactionnel peut être désactivé par l'injection d'ethyl-2-hexanol utilisé comme inhibiteur. Le mélange peut être réalisé en utilisant un mélangeur dynamique en ligne (non représenté dans la figure 1). En particulier, le milieu réactionnel issu du réacteur peut être détendu dans un premier ballon flash à une pression de préférence de 2,4 MPa. Ledit milieu réactionnel détendu peut ensuite être vaporisé par chauffage jusqu'à une température de préférence de 175°C. Cette vaporisation est effectuée dans un échangeur de chaleur. La phase vapeur est alors séparée de la phase liquide dans un ballon. La phase vapeur est envoyée vers la section de séparation (3). La phase liquide résultant du premier ballon flash est détendue dans un deuxième ballon flash à une pression de préférence de 1,1 MPa. Ladite phase liquide détendue est ensuite vaporisée par chauffage jusqu'à une température de préférence de 175°C. Cette vaporisation est dans réalisée dans un échangeur de chaleur. La phase vapeur est alors séparée de la phase liquide dans un ballon. La phase vapeur est envoyée vers la section de séparation 3 et la phase liquide résultant du deuxième ballon flash est détendue à une pression de préférence de 0,2 MPa. Ladite phase liquide détendue est ensuite vaporisée par chauffage jusqu'à une température de préférence de 200°C. Cette vaporisation est effectuée de préférence dans un évaporateur à couche mince. Le catalyseur désactivé ainsi que les sous-produits lourds séparés dans la section de désactivation et d'évaporation (2) sont pompés et envoyés vers un incinérateur.

### Section de séparation (3)

Dans le cadre général de l'invention qui concerne un procédé d'oligomérisation des oléfines, l'étape de séparation est avantageusement réalisée dans la section de séparation (3) qui sépare les différents produits formés dans la réaction d'oligomérisation des oléfines.

Les oléfines oligomères issues de l'oligomérisation de l'éthylène possèdent un poids moléculaire plus important que l'éthylène n'ayant pas réagi. D'une façon générale, l'éthylène n'ayant pas réagi présente une température d'ébullition plus basse que celle des oléfines oligomères issues de la réaction. Tout moyen de séparation connu de l'homme du métier mettant à profit ces écarts de volatilité et de poids moléculaire entre les produits à séparer peut être mis en œuvre. Avantageusement selon l'invention, les moyens de séparation mis en œuvre dans l'étape de séparation dans la section de séparation (3) sont des colonnes de distillation de tout type.

Dans le cas d'un procédé d'un procédé de dimérisation de l'éthylène on sépare de préférence uniquement l'éthylène non réagi (flux 306) du butene-1 (flux 301) et des produits hydrocarbonés C6+ (flux 304).

Dans le cas d'un procédé d'un procédé de trimérisation de l'éthylène on sépare de préférence l'éthylène non réagi (flux 306), les produits d'intérêt tels que l'hexène-1 et éventuellement du diluant (flux 301 et éventuellement 302, 303 ...) et les fractions de composés plus lourds (flux 304).

La section de séparation (3) est avantageusement munie de plusieurs colonnes à distiller (non représenté dans la figure 1).

Avantageusement selon l'invention, l'éthylène non réagi séparé dans l'étape de séparation dans la section de séparation (3) est recyclé au réacteur (flux 306).

Dans le cas particulier d'un procédé de trimérisation de l'éthylène, la section de séparation (3) peut comprendre au moins quatre colonnes de distillation disposées de la façon suivante: une première colonne de distillation dans laquelle l'éthylène non converti est séparé dans une fraction de tête du reste des composés dans la fraction de fond ; une deuxième colonne de distillation dans laquelle on sépare la fraction de fond issue de la première colonne en une fraction de tête comprenant de l'hexène-1 et du butène-1 et une fraction de fond. Ladite fraction de fond est principalement composée du diluant ; une troisième colonne de distillation dans laquelle on sépare la fraction de tête de la deuxième distillation en une fraction de tête comprenant principalement du butène-1 et en une fraction de fond comprenant principalement de l'hexène-1 produit de la réaction ; une quatrième colonne dans laquelle on sépare la fraction de fond de la deuxième distillation en une fraction de tête comprenant principalement du diluant qui peut être recyclé au réacteur et en une fraction de fond comprenant les produits plus lourds notamment en C8+ (à 8 atomes de carbones ou plus). Le catalyseur désactivé ainsi que les sous-produits lourds sont évacués.

Dans une variante du procédé selon l'invention, le solvant de nettoyage est constitué du diluant. Dans une variante du procédé selon l'invention, le solvant de nettoyage est constitué de la fraction de fond comprenant les produits plus lourds notamment en C8+ issus du fond de la quatrième colonne. Dans une variante du procédé selon l'invention, le solvant de nettoyage est constitué du produit de fond issu de la deuxième colonne.

### Section de nettoyage de l'échangeur de chaleur 12a/12b

Conformément à l'invention, le réacteur utilisé dans la section réactionnelle 1 est muni d'une boucle de refroidissement (100a/b, 101a/b, 102a/b) au moyen de laquelle on fait circuler au moins le milieu réactionnel à travers au moins deux échangeurs de chaleur pour permettre d'extraire la chaleur générée par la réaction d'oligomérisation afin d'éviter une élévation incontrôlée de la température du milieu réactionnel dans le réacteur.

Selon l'invention, les échangeurs de chaleur (12a, 12b) utilisés sont permutables et sont aptes à être connectés à au moins une boucle de refroidissement, de sorte que lorsqu' au moins un échangeur de chaleur est en fonctionnement connecté à au moins une boucle de refroidissement, l'autre échangeur de chaleur est déconnecté et soumis à une étape de nettoyage au moyen d'un dispositif de nettoyage dans lequel on fait circuler en boucle un solvant apte à nettoyer ledit échangeur de chaleur, ledit dispositif de nettoyage comprenant :
- un ballon d'inventaire du solvant de nettoyage (40),
- un échangeur de chaleur (43) permettant de chauffer le solvant de nettoyage à une température supérieure à 130°C, de manière préférée supérieure à 150°C, de manière plus préférée supérieure à 170°C, de manière encore plus préférée supérieure à 180°C de façon à permettre la dissolution du polymère déposé dans l'échangeur de chaleur déconnecté,
- une pompe de recirculation (42) permettant la circulation en boucle du solvant de nettoyage dans le dispositif de nettoyage entre le ballon d'inventaire du solvant de nettoyage (40), l'échangeur de chaleur (43) du solvant de nettoyage et l'échangeur de chaleur déconnecté que l'on souhaite nettoyer.

Le nettoyage est réalisé en circulant le solvant chaud à l'aide de la pompe de recirculation (42) via les conduites 402, 403, 404 ,405.

Selon l'invention, la pression du ballon (40) d'inventaire de solvant de nettoyage est de préférence fixée de façon à maintenir le solvant de nettoyage sous forme liquide. Cette pression est de préférence comprise entre 0,5 et 3 MPa.

Le procédé selon l'invention peut mettre en œuvre plusieurs échangeurs de chaleur (12a, 12b) aptes à être connectés à au moins une boucle de refroidissement permettant d'extraire la chaleur générée par la réaction d'oligomérisation. De préférence 2 ou 3 ou 4 ou 5 échangeurs de chaleur peuvent être mis en œuvre. La figure 1 dispose de deux boucles de refroidissement comprenant chacune un échangeur de chaleur mises en œuvre en alternance. Les vannes 15a/16a sont ouvertes et connectées au réacteur (10) lorsque l'échangeur de chaleur (12a) est en fonctionnement avec la boucle de refroidissement (100a, 101a, 102a). Pendant ce temps, les vannes 15b/16b sont fermées isolant ainsi la seconde boucle de refroidissement (100b, 101b, 102b) du réacteur 10 et permettre son nettoyage par le dispositif de nettoyage.

Le solvant de nettoyage est avantageusement choisi parmi les hydrocarbures saturés comme le butane, l'isobutane, le pentane, le cyclohexane, le méthylecyclohexane, le n-hexane, l'heptane, l'octane, le décane, le dodécane ; les hydrocarbures insaturés comme une monooléfine ou une dioléfine comportant par exemple de 4 à 20 atomes de carbone ; les hydrocarbures aromatiques tels que le benzène, le toluène, l'ortho le méta ou le paraxylène, le cumène, le mésitylène, l'éthylbenzène ; une coupe essence, diesel ou kérosène; des isoparaffines telles que l'Isopar E ou C ; utilisés seuls ou en mélange. Le solvant de nettoyage peut être issu du procédé lui-même, notamment de la section de séparation (3). De préférence, au moins une partie du solvant de nettoyage, voire la totalité du solvant de nettoyage provient de l'étape de séparation de la section de séparation 3 (flux 305). De préférence, au moins une partie du solvant de nettoyage voire la totalité du solvant de nettoyage provient des fractions de composés plus lourds et/ou d'un éventuel diluant séparés de l'étape de séparation de la section de séparation 3.

Dans une variante de l'invention un filtre (39) pourra être implémenté sur la boucle de circulation de solvant de nettoyage (402, 403, 404, 405). Ce filtre permettra de capter d'éventuels morceaux de polymères qui se seraient décollés de l'échangeur de chaleur que l'on nettoie sans être totalement dissous.

Selon l'invention, au moins une partie du solvant de nettoyage chargé issu de la boucle de solvant du dispositif de nettoyage (406) est envoyée dans l'étape d'évaporation des produits dans la section d'évaporation (2).

Par exemple, une fois le polymère dissous dans le solvant de nettoyage, au moins une partie du solvant de nettoyage chargé peut être dirigée vers l'étape d'évaporation des produits, notamment dans la section d'évaporation (2) (voir les conduites 406, 407, 211, 212, 210 et 213 dans la figure 1).

Selon l'invention, une fois le polymère dissous dans le solvant de nettoyage, au moins une partie du solvant de nettoyage chargé (406-407) est envoyée en mélange avec une partie de l'effluent réactionnel 103 dans la section d'évaporation (2), soit en amont (flux 213), soit en aval (flux 211) de l'échangeur de chaleur (20) lorsque cet échangeur de chaleur est présent.

Selon la présente divulgation, une fois le polymère dissous dans le solvant de nettoyage, au moins une partie du solvant de nettoyage chargé (406-407) pourrait être envoyée en mélange avec la phase liquide (202) issue du ballon flash (21) (flux 212) mis en œuvre dans la section d'évaporation (2).

Selon la présente divulgation, une fois le polymère dissous dans le solvant de nettoyage, au moins une partie du solvant de nettoyage chargé (406-407) pourrait être envoyée en mélange avec la phase liquide 204 issue du vaporiseur ou de l'évaporateur à film mince (22) (flux 210) mis en œuvre dans la section d'évaporation (2).

La concentration en polymères et oligomères lourds dans le solvant de nettoyage chargé est avantageusement inférieure à 5% poids, et de façon préférée inférieure à 1% poids par rapport au solvant de nettoyage.

Dans une variante de l'invention, une fois le polymère dissous dans le solvant de nettoyage, au moins une partie du solvant de nettoyage chargé, de préférence prélevée du flux (406) (408), est détendue et dirigée vers une étape de vaporisation partielle dans au moyen d'un ballon flash (46) permettant de produire au moins une phase vapeur (409) et au moins une phase liquide (410). La vaporisation partielle résultant de cette détente présente deux avantages. Tout d'abord, elle permet de générer une phase vapeur dont la teneur en polymères, oligomères lourds et catalyseur est suffisamment faible pour permettre de la diriger directement après condensation(s) éventuelle(s) dans l'échangeur (45) (ligne 409, 412) vers la section de séparation (3) du procédé. Au moins une partie de cette phase vapeur peut aussi, après condensation(s) dans l'échangeur 45, être collectée dans le ballon de recette 41 puis renvoyée au ballon d'inventaire de solvant 40 (flux 413, 414) afin de servir de nouveau au nettoyage de l'échangeur selon le procédé de l'invention.

Par ailleurs cette vaporisation partielle permet d'abaisser la température du flux 410/411 de la phase liquide récupérée du ballon flash 46 de façon à pouvoir la réinjecter, de préférence en mélange avec le flux (407), dans la section d'évaporation (2) sans nécessiter de refroidissement. En effet une température trop élevée dans les sections en aval du réacteur avant séparation totale du catalyseur même désactivé peut amener une dégradation des produits. Dans une telle mise en œuvre, une pompe de recirculation (44) peut être implémentée entre les flux 410 et 411. On peut également implémenter un échangeur de chaleur dans la conduite 407 (47).

La vaporisation partielle du solvant de nettoyage chargé est réalisée de façon préférentielle par une détente car l'utilisation d'un échangeur de chaleur comporterait un fort risque d'encrassement de ce dernier.

Selon une autre variante du procédé de l'invention, au moins une partie du solvant de nettoyage provient de l'étape de séparation de la section de séparation (3) (flux 305). De préférence, au moins une partie du solvant de nettoyage provient des fractions de composés plus lourds et/ou d'un éventuel diluant séparés de l'étape de séparation, évitant alors la nécessité d'un apport extérieur en solvant de nettoyage. De préférence, le solvant de nettoyage est exclusivement constitué des produits hydrocarbonés plus lourds, récupérés de la section de séparation (3) (flux 304). Une fraction des produits hydrocarbonés plus lourds peut être dirigée (305) vers le ballon 40 d'inventaire de solvant de nettoyage. De cette façon aucun appoint extérieur en solvant de solvant n'est requis. La conduite 305 peut être munie d'un échangeur de chaleur (48).

La présente divulgation concerne également une installation mise en œuvre dans le procédé selon l'invention.

L'installation selon la présente divulgation comprend :
- une section réactionnelle (1) comprenant un réacteur (10),
- une section d'évaporation (2) de l'effluent réactionnel,
- une section de séparation (3),
ledit réacteur étant muni d'au moins une boucle de refroidissement (100a/b, 101a/b, 102a/b) au moyen de laquelle on fait circuler le milieu réactionnel à travers au moins deux échangeurs de chaleur permutables aptes à être connectés à au moins une boucle de refroidissement, de sorte que lorsque au moins un échangeur de chaleur est en fonctionnement connecté à au moins une boucle de refroidissement, l'autre échangeur de chaleur déconnecté est soumis à une étape de nettoyage au moyen d'un dispositif de nettoyage dans lequel on fait circuler un solvant apte à nettoyer ledit échangeur de chaleur, le dispositif de nettoyage comprenant :
- un ballon d'inventaire du solvant de nettoyage (40),
- un échangeur de chaleur (43) permettant de chauffer le solvant de nettoyage à une température supérieure à 130°C de façon à permettre la dissolution du polymère déposé dans l'échangeur de chaleur déconnecté,
- une pompe de recirculation (42) permettant la circulation en boucle du solvant de nettoyage dans le dispositif de nettoyage entre le ballon d'inventaire du solvant de nettoyage (40), l'échangeur de chaleur (43) du solvant de nettoyage et l'échangeur de chaleur déconnecté que l'on souhaite nettoyer.

Selon la présente divulgation, le dispositif de nettoyage est connecté à la section d'évaporation (2) par au moins un conduit permettant d'envoyer au moins une partie du solvant de nettoyage dans ladite section d'évaporation (2) de l'effluent réactionnel.

### Exemple 1: Application du procédé selon l'invention à un procédé de dimérisation de l'éthylène

La production de butène-1 est réalisée par la dimérisation catalytique homogène en phase liquide d'éthylène. Le dispositif selon l'invention tel que décrit dans la figure 1 est mis en œuvre. Le catalyseur est obtenu par interaction de titanate de n-butyle, du tétrahydrofurane et du triéthylaluminium tel que décrit dans l'exemple 1 du brevet EP135441.

Au cours de la réaction de dimérisation de l'éthylène, la pression du réacteur 10 est de 2,2 MPa et la température de 53°C.

Le réacteur est refroidi à l'aide de deux boucles de refroidissement comportant chacun un échangeur de chaleur (12a et 12b). L'échangeur de chaleur 12b permutable est déconnecté après une période de fonctionnement de 2 mois. Dans l'exemple il est considéré que l'on procède au nettoyage de cet échangeur 12b tandis que l'échangeur 12a est en opération.

L'effluent réactionnel 103 contient de l'éthylène, le butene-1 produit ainsi que certains produits hydrocarbonés en C6 et C8+ (principalement des octènes) formés au cours de réactions secondaires dans le réacteur (10). On introduit l'effluent réactionnel 103 dans la section (2) de désactivation et d'évaporation. L'effluent réactionnel (103) est mis en contact avec de la décylamine employée comme inhibiteur du catalyseur. La section (2) comprend un échangeur de chaleur (20) dans laquelle l'effluent réactionnel (103) est vaporisé à une pression de 2,1 MPa et à la température de 80°C puis envoyé dans le ballon flash (21) afin de séparer une phase gazeuse (207) contenant essentiellement du butène-1 et de l'éthylène et d'une seconde phase liquide (202) contenant le catalyseur désactivé. On dirige alors la phase liquide (202) issue du ballon flash (21) dans un vaporiseur à film mince (22) dans lequel elle est évaporée à une pression sensiblement identique à celle du ballon flash (21), dans des conditions permettant la séparation d'une phase gazeuse (203) contenant de l'éthylène, du butène-1, ainsi que des produits hydrocarbonés plus lourds (C6+), et d'une phase liquide enrichie en catalyseur désactivé concentré (204). Cette phase liquide (204) obtenue est détendue et flashée dans un ballon flash (23) générant une phase vapeur (205) sensiblement dépourvue de polymère et catalyseur désactivé et une phase liquide (206) concentrée et polymères et catalyseur désactivé. Les compositions des flux issus de la section de désactivation et d'évaporation sont décrites dans le tableau 1 ci-dessous.

| % poids | Effluent réactionnel (103) | phase gazeuse (207) | phase gazeuse (203) | phase gazeuse (205) | liquide 3ème flash (206) |
|---|---|---|---|---|---|
| Méthane | 0,47 | 0,92 | 0,11 | 0,00 | 0,00 |
| Ethylene | 12,03 | 20,69 | 5,41 | 0,00 | 0,00 |
| Ethane | 0,90 | 1,46 | 0,48 | 0,00 | 0,00 |
| Butene-1 | 80,84 | 74,59 | 86,03 | 83,33 | 27,27 |
| N-Butane | 0,12 | 0,10 | 0,13 | 0,00 | 0,00 |
| Hexènes | 5,08 | 2,10 | 7,21 | 16,67 | 27,27 |
| Hexane | 0,30 | 0,10 | 0,42 | 0,00 | 4,55 |
| C8+ | 0,16 | 0,03 | 0,21 | 0,00 | 9,09 |
| triéthylaluminium | 0,00 | 0,00 | 0,00 | 0,00 | 0,00 |
| Décylamine | 0,01 | 0,00 | 0,00 | 0,00 | 4,55 |
| Catalyseur désactivé | 0,09 | 0,00 | 0,00 | 0,00 | 27,27 |

Les différentes phases vapeur 207 et 203 issues du ballon flashs (21) et du vaporiseur à film mince (22), sensiblement exemptes de catalyseur désactivé, sont condensées sous la forme d'un produit liquide qui peut être amené dans une section de distillation correspondant à la section de séparation (3) dans laquelle les différents produits seront séparés. Dans la section de distillation (3), on réalise la séparation de l'éthylène d'une part et du butene-1 et des composées plus lourds d'autre part dans une seconde colonne à distiller (non représentée). La fraction enrichie en éthylène est recyclé au réacteur (flux 306). On réalise la séparation entre le butene-1 (flux 301) et les composés plus lourds (C6+, flux 304) dans une seconde colonne à distiller (non représentée).

Pour le nettoyage de l'échangeur 12b, le dispositif selon l'invention est mis en œuvre. Le n-hexane est utilisé comme solvant de nettoyage. Une circulation (lignes 402, 403, 404, 405) du solvant de nettoyage est établie à l'aide de la pompe (42) entre le ballon (40) d'inventaire de solvant et l'échangeur 12b à nettoyer. Un échangeur de chaleur 43 sur cette boucle permet de chauffer le solvant à une température de 180°C.

La pression du ballon (40) d'inventaire de solvant de nettoyage (n-hexane) est d'environ 1,4 MPa absolu de façon à le maintenir sous forme liquide. Une fois le nettoyage terminé, le solvant chargé en polymère et résidus de catalyseurs est dirigé via les lignes 406, 407 et 211 vers le ballon flash (21).

Le procédé est mis en œuvre sans arrêt et permet d'éviter d'ouvrir des équipements, notamment les échangeurs de chaleur pour les nettoyer, ce qui représente un gain en main d'œuvre et évite les risques d'accident.

## Revendications

1. Procédé d'oligomérisation de l'éthylène en alphaoléfines comprenant
- une étape d'oligomérisation de l'éthylène dans une section réactionnelle (1) comprenant un réacteur (10) en présence d'un catalyseur, éventuellement un diluant,
- une étape de désactivation du catalyseur contenu dans l'effluent réactionnel,
- une étape d'évaporation des produits contenus dans l'effluent réactionnel, pour les séparer du catalyseur désactivé, réalisée dans une section d'évaporation (2),
- une étape de séparation des produits contenus dans l'effluent réactionnel dans une section de séparation (3),
ledit réacteur étant muni d'au moins une boucle de refroidissement (100a/b, 101a/b, 102a/b) au moyen de laquelle on fait circuler le milieu réactionnel à travers au moins deux échangeurs de chaleur permutables aptes à être connectés à au moins une boucle de refroidissement, de sorte que lorsque au moins un échangeur de chaleur est en fonctionnement connecté à au moins boucle de refroidissement, l'autre échangeur de chaleur déconnecté est soumis à une étape de nettoyage du polymère déposé dans l'échangeur de chaleur déconnecté au moyen d'un dispositif de nettoyage dans lequel on fait circuler en boucle un solvant apte à nettoyer ledit échangeur de chaleur, le dispositif de nettoyage comprenant :
- un ballon d'inventaire du solvant de nettoyage (40),
- un échangeur de chaleur (43) permettant de chauffer le solvant de nettoyage à une température supérieure à 130°C de façon à permettre la dissolution du polymère déposé dans l'échangeur de chaleur déconnecté,
- une pompe de recirculation (42) permettant la circulation en boucle du solvant de nettoyage dans le dispositif de nettoyage entre le ballon d'inventaire du solvant de nettoyage (40), l'échangeur de chaleur (43) du solvant de nettoyage et l'échangeur de chaleur déconnecté que l'on souhaite nettoyer,
dans lequel au moins une partie du solvant de nettoyage chargé (406, 407) est envoyée en mélange avec une partie de l'effluent réactionnel (103) dans la section d'évaporation (2), soit en amont (213), soit en aval (211) d'un échangeur de chaleur (20) de ladite section d'évaporation (2) lorsque cet échangeur de chaleur est présent.

2. Procédé selon la revendication 1 dans lequel la pression du ballon (40) d'inventaire de solvant de nettoyage est comprise entre 0,5 et 3 MPa.

3. Procédé selon l'une quelconque des revendications précédentes dans lequel le milieu réactionnel (103) est circulé dans un échangeur de chaleur (20) dans lequel il est vaporisé dans la section d'évaporation (2).

4. Procédé selon l'une des revendications précédentes dans lequel l'éthylène non réagi séparé dans l'étape de séparation dans la section de séparation (3) est recyclé au réacteur (flux (306)).

5. Procédé selon l'une des revendications précédentes dans lequel l'échangeur de chaleur (43) chauffe le solvant de nettoyage à une température supérieure à 150°C de façon à permettre la dissolution du polymère déposé dans l'échangeur de chaleur déconnecté.

6. Procédé selon l'une des revendications précédentes dans lequel la concentration en polymères et oligomères lourds dans le solvant chargé est inférieure à 5% poids par rapport au solvant de nettoyage.

7. Procédé selon l'une des revendications précédentes dans lequel le solvant de nettoyage est choisi parmi les hydrocarbures saturés ; les hydrocarbures insaturés ; les hydrocarbures aromatiques ; une coupe essence, diesel ou kérosène ; des isoparaffines ; utilisés seuls ou en mélange.

8. Procédé selon l'une des revendications précédentes dans lequel au moins une partie du solvant de nettoyage provient de l'étape de séparation de la section de séparation (3) (flux (305)).

9. Procédé selon l'une des revendications précédentes dans lequel, au moins une partie du solvant de nettoyage provient des fractions de composés plus lourds et/ou d'un éventuel diluant séparés de l'étape de séparation.

10. Procédé selon l'une des revendications précédentes dans lequel un filtre (39) est implémenté sur la boucle de circulation de solvant de nettoyage (402, 403, 404, 405).

11. Procédé selon l'une des revendications précédentes dans lequel une fois le polymère dissous dans le solvant de nettoyage, au moins une partie du solvant de nettoyage chargé est détendue et dirigée vers une étape de vaporisation partielle dans un ballon flash (46) permettant de produire au moins une phase vapeur (408) et au moins une phase liquide (410).

12. Procédé selon l'une des revendications 1 à 11 dans lequel le procédé d'oligomérisation de l'éthylène est un procédé de dimérisation de l'éthylène en butène-1.

13. Procédé selon l'une des revendications 1 à 11 dans lequel le procédé d'oligomérisation de l'éthylène est un procédé de trimérisation de l'éthylène en hexène-1.

14. Procédé selon l'une des revendications 1 à 11 dans lequel le procédé d'oligomérisation de l'éthylène est un procédé de tétramérisation de l'éthylène en octène-1.

## Patentansprüche

1. Verfahren zur Oligomerisierung von Ethylen zu alpha-Olefinen, umfassend
- einen Schritt des Oligomerisierens des Ethylens in einem Reaktionsabschnitt (1), der einen Reaktor (10) in Gegenwart eines Katalysator, möglicherweise ein Verdünnungsmittel, umfasst,
- einen Schritt des Deaktivierens des Katalysators, welcher in dem Reaktionsstoffstrom enthalten ist,
- einen Schritt des Verdampfens der Produkte, welche in dem Reaktionsstoffstrom enthalten sind, um sie von dem deaktivierten Katalysator abzutrennen, wobei er in einem Verdampfungsabschnitt (2) durchgeführt wird,
- einen Schritt des Auftrennens der Produkte, welche in dem Reaktionsstoffstrom enthalten sind, in einem Auftrennungsabschnitt (3),
wobei der Reaktor mit mindestens einer Kühlschleife (100a/b, 101a/b, 102a/b) versehen ist, mittels welcher das Reaktionsmilieu durch mindestens zwei wechselseitig verwendbare Wärmetauscher geleitet wird, die an mindestens eine Kühlschleife angeschlossen werden können, sodass im Betrieb mindestens ein Wärmetauscher an mindestens eine Kühlschleife angeschlossen ist, wobei der andere Wärmetauscher nicht angeschlossen ist und einem Schritt unterzogen wird, der darin besteht, mittels einer Reinigungsvorrichtung, in welcher ein Lösungsmittel umgewälzt wird, mittels dessen der nicht angeschlossene Wärmetauscher gereinigt werden kann, das Polymer reinigend zu entfernen, welches sich in diesem Wärmetauscher abgelagert hat, wobei die Reinigungsvorrichtung Folgendes umfasst:
- einen Vorratskolben für das reinigende Lösungsmittel (40),
- einen Wärmetauscher (43), der es ermöglicht, das reinigende Lösungsmittel auf eine Temperatur von mehr als 130 °C zu erhitzen, sodass das Polymer, welches sich in dem nicht angeschlossenen Wärmetauscher abgelagert hat, aufgelöst werden kann,
- eine Umwälzpumpe (42), die es ermöglicht, das reinigende Lösungsmittel in der Reinigungsvorrichtung zwischen dem Vorratsbehälter für das reinigende Lösungsmittel (40), dem Wärmetauscher (43) für das reinigende Lösungsmittel und dem nicht angeschlossenen Wärmetauscher, welche gereinigt werden soll, umzuwälzen,
wobei mindestens eine Teilmenge des befrachteten reinigenden Lösungsmittels (406, 407) in Mischung mit einer Teilmenge des Reaktionsstoffstroms (103) dem Verdampfungsabschnitt (2) zugeführt wird, entweder stromaufwärts (213) oder stromabwärts (211) eines Wärmetauschers (20) des Verdampfungsabschnitts (2), sofern ein solcher Wärmetauscher vorliegt.

2. Verfahren nach Anspruch 1, wobei der Druck in dem Vorratsbehälter (40) für das reinigende Lösungsmittel im Bereich von 0,5 bis 3 MPa liegt.

3. Verfahren nach einem beliebigen der vorhergehenden Ansprüche, wobei das Reaktionsmilieu (103) in einen Wärmetauscher (20) geleitet wird, um dort im Verdampfungsabschnitt (2) verdampft zu werden.

4. Verfahren nach einem der vorhergehenden Ansprüche, wobei das nicht umgesetzte Ethylen, welches in dem Auftrennungsschritt im Auftrennungsabschnitt (3) abgetrennt wurde, in den Reaktor zurückgeführt wird (Stoffstrom (306)).

5. Verfahren nach einem der vorhergehenden Ansprüche, wobei der Wärmetauscher (43) das reinigende Lösungsmittel auf eine Temperatur von mehr als 150 °C erhitzt, sodass das Polymer, welches sich in dem nicht angeschlossenen Wärmetauscher abgelagert hat, aufgelöst werden kann.

6. Verfahren nach einem der vorhergehenden Ansprüche, wobei die Konzentration an Polymeren und schwerflüchtigen Oligomeren in dem befrachteten Lösungsmittel weniger als 5 Gewichts-% beträgt, bezogen auf das reinigende Lösungsmittel.

7. Verfahren nach einem der vorhergehenden Ansprüche, wobei das reinigende Lösungsmittel aus den gesättigten Kohlenwasserstoffen; den ungesättigten Kohlenwasserstoffen; den aromatischen Kohlenwasserstoffen; einer Benzin-, Diesel- oder Kerosinfraktion; Isoparaffinen ausgewählt ist; wobei diese allein oder in Mischung verwendet werden.

8. Verfahren nach einem der vorhergehenden Ansprüche, wobei mindestens eine Teilmenge des reinigenden Lösungsmittels aus dem Auftrennungsschritt des Auftrennungsabschnitts (3) stammt (Stoffstrom (305)).

9. Verfahren nach einem der vorhergehenden Ansprüche wobei mindestens eine Teilmenge des reinigenden Lösungsmittels aus Fraktionen von relativ schwerflüchtigen Verbindungen und/oder gegebenenfalls aus einem Verdünnungsmittel stammt, wobei diese im Auftrennungsschritt abgetrennt wurden.

10. Verfahren nach einem der vorhergehenden Ansprüche, wobei die Umwälzschleife des reinigenden Lösungsmittels (402, 403, 404, 405) mit einem Filter (39) versehen ist.

11. Verfahren nach einem der vorhergehenden Ansprüche, wobei mindestens eine Teilmenge des befrachteten reinigenden Lösungsmittels, nachdem sich das Polymer in dem reinigenden Lösungsmittel aufgelöst hat, entspannt und einem Schritt des teilweisen Verdampfens in einem Entspannungsverdampfungskolben (46) zugeführt wird, wodurch es ermöglicht wird, mindestens eine Dampfphase (408) und mindestens eine Flüssigphase (410) zu erzeugen.

12. Verfahren nach einem der Ansprüche 1 bis 11, wobei es sich bei dem Verfahren zur Oligomerisierung von Ethylen um ein Verfahren zur Dimerisierung von Ethylen zu 1-Buten handelt.

13. Verfahren nach einem der Ansprüche 1 bis 11, wobei es sich bei dem Verfahren zur Oligomerisierung von Ethylen um ein Verfahren zur Trimerisierung von Ethylen zu 1-Hexen handelt.

14. Verfahren nach einem der Ansprüche 1 bis 11, wobei es sich bei dem Verfahren zur Oligomerisierung von Ethylen um ein Verfahren zur Tetramerisierung von Ethylen zu 1-Octen handelt.

## Claims

1. A process for the oligomerization of ethylene into alpha-olefins, comprising:
- a step for oligomerization of the ethylene in a reaction section (1) comprising a reactor (10), in the presence of a catalyst, and optionally a diluent,
- a step for deactivation of the catalyst contained in the reaction effluent,
- a step for evaporation of the products contained in the reaction effluent in order to separate them from the deactivated catalyst, carried out in an evaporation section (2),
- a step for separation of the products contained in the reaction effluent in a separation section (3),
said reactor being provided with at least one cooling loop (100a/b, 101ab, 102a/b), by means of which the reaction medium is caused to move through at least two switchable heat exchangers which are capable of being connected to at least one cooling loop in a manner such that when at least one heat exchanger is operatively connected to at least one cooling loop, the other disconnected heat exchanger undergoes a step for cleaning the polymer deposited in the disconnected heat exchanger by means of a cleaning device in which a solvent which is capable of cleaning said heat exchanger is caused to move in a loop, the cleaning device comprising:
- a storage drum (40) for the cleaning solvent,
- a heat exchanger (43) for heating the cleaning solvent to a temperature above 130°C in a manner such as to allow the polymer deposited in the disconnected heat exchanger to be dissolved;
- a recirculation pump (42) for moving the cleaning solvent in a loop in the cleaning device between the cleaning solvent storage drum (40), the heat exchanger (43) for the cleaning solvent and the disconnected heat exchanger which is to be cleaned,
in which at least a portion of the charged cleaning solvent (406, 407) is sent to the evaporation section (2) as a mixture with a portion of the reaction effluent (103) either upstream (213) or downstream (211) of a heat exchanger (20) of said evaporation section (2) when this heat exchanger is present.

2. The process as claimed in claim 1, in which the pressure of the cleaning solvent storage drum (40) is between 0.5 and 3 MPa.

3. The process as claimed in one of the preceding claims, in which the reaction medium (103) is moved in a heat exchanger (20) in which it is vaporized in the evaporation section (2).

4. The process as claimed in one of the preceding claims, in which the unreacted ethylene separated in the separation step in the separation section (3) is recycled to the reactor (stream (306)).

5. The process as claimed in one of the preceding claims, in which the heat exchanger (43) heats the cleaning solvent to a temperature of more than 150°C in a manner such as to dissolve the polymer deposited in the disconnected heat exchanger.

6. The process as claimed in one of the preceding claims, in which the concentration of polymers and heavy oligomers in the charged solvent is less than 5% by weight with respect to the cleaning solvent.

7. The process as claimed in one of the preceding claims, in which the cleaning solvent is selected from saturated hydrocarbons; unsaturated hydrocarbons; aromatic hydrocarbons; a gasoline, diesel or kerosene cut; and isoparaffins; used alone or as a mixture.

8. The process as claimed in one of the preceding claims, in which at least a portion of the cleaning solvent comes from the step for separation in the separation section (3) (stream (305)).

9. The process as claimed in one of the preceding claims, in which at least a portion of the cleaning solvent originates from heavier compound fractions and/or from an optional diluent separated from the separation step.

10. The process as claimed in one of the preceding claims, in which a filter (39) is employed on the loop for moving cleaning solvent (402, 403, 404, 405) .

11. The process as claimed in one of the preceding claims, in which, once the polymer has been dissolved in the cleaning solvent, at least a portion of the charged cleaning solvent is depressurized and directed towards a partial vaporization step in a flash drum (46), in order to produce at least one vapour phase (408) and at least one liquid phase (410).

12. The process as claimed in one of claims 1 to 11, in which the ethylene oligomerization process is a process for the dimerization of ethylene to 1-butene.

13. The process as claimed in one of claims 1 to 11, in which the ethylene oligomerization process is a process for the trimerization of ethylene to 1-hexene.

14. The process as claimed in one of claims 1 to 11, in which the ethylene oligomerization process is a process for the tetramerization of ethylene to 1-octene.
